**Europäisches Patentamt**

**European Patent Office**

**Office européen des brevets**

(19)

(11) Veröffentlichungsnummer : **0 431 567 B1**

(12)

# EUROPÄISCHE PATENTSCHRIFT

(45) Veröffentlichungstag der Patentschrift :
**08.03.95 Patentblatt 95/10**

(51) Int. Cl.$^6$ : **G01N 33/574**

(21) Anmeldenummer : **90123247.0**

(22) Anmeldetag : **04.12.90**

(54) Verfahren zum Nachweis eines kleinzelligen Bronchial-Karzinoms.

(30) Priorität : **05.12.89 DE 3940209**

(43) Veröffentlichungstag der Anmeldung :
**12.06.91 Patentblatt 91/24**

(45) Bekanntmachung des Hinweises auf die
Patenterteilung :
**08.03.95 Patentblatt 95/10**

(84) Benannte Vertragsstaaten :
**AT BE CH DE DK ES FR GB GR IT LI LU NL SE**

(56) Entgegenhaltungen :
**WO-A-88/07551
PATH. RES. PRACT., Band 183, 1988; K. KA-
YSER et al., Seiten 412-417
THE EMBO JOURNAL, Band 6, Nr. 11, 1987;
R.E. LEUBE et al., Seiten 3261-3268**

(73) Patentinhaber : **BOEHRINGER MANNHEIM
GMBH
D-68298 Mannheim (DE)**

(72) Erfinder : **Bodenmüller, Heinz, Dr.
Zugspitzstrasse 1
W-8132 Tutzing (DE)**
Erfinder : **Dessauer, Andreas, Dr.
Herre-Strasse 1
W-8132 Tutzing (DE)**

(74) Vertreter : **Huber, Bernhard, Dipl.-Chem. et al
PatentanwälteH. Weickmann, Dr. K.
FinckeF.A. Weickmann, B. HuberDr. H. Liska,
Dr. J. Prechtel, Dr. B.
BöhmPostfach 86 08 20
D-81635 München (DE)**

## Beschreibung

Die Erfindung betrifft ein Verfahren zum Nachweis eines kleinzelligen Bronchial-Karzinoms durch Bestimmung von Synaptophysin.

Unter den Krebserkrankungen sind maligne Tumoren der Lunge und des Atemwegbereiches besonders häufig vertreten und breiten sich durch invasives Wachstum sowohl regional als auch via Fernmetastasierung aus. Klinisch wird hierbei unter vier verschiedenen Haupttumorarten unterschieden und zwar zwischen dem kleinzelligen Bronchial-Karzinom (SCLC, small cell lung carcinoma), dem Plattenepithel-Karzinom, dem Adeno-Karzinom und dem großzelligen anaplastischen Karzinom, wobei die drei letztgenannten Arten üblicherweise zum Begriff der "nicht kleinzellige Tumoren" zusammengefaßt werden. Da kleinzellige Bronchial-Karzinome einerseits besonders früh metastasieren und dazu noch ein ausgesprochen schnelles Tumorwachstum aufweisen, andererseits aber im Gegensatz zu den nicht kleinzelligen Bronchial-Karzinomen eine hohe Sensibilität gegenüber Radio- und Chemotherapie aufweisen, ist ein frühzeitiges Erkennen dieses Tumors und seine Unterscheidung von anderen Tumorarten für die Prognose dieser Erkrankung entscheidend.

Der Erfindung lag nun die Aufgabe zugrunde, einen Nachweis für das Auftreten von kleinzelligen Bronchial-Karzinomen bereitzustellen, ohne daß dabei Gewebsproben von Patienten entnommen werden müssen.

Diese Aufgabe wird erfindungsgemäß nun dadurch gelöst, daß man Synaptophysin, seine Oligomere und/oder seine Bruchstücke in Körperflüssigkeiten bestimmt. Es wurde nämlich überraschenderweise gefunden, daß das sonst fest an die Membran gebundene Protein Synaptophysin, seine Oligomere und/oder seine immunologisch erkennbaren Bruchstücke sich bei Patienten mit kleinzelligen Bronchial-Karzinomen in Körperflüssigkeiten nachweisen läßt.

Synaptophysin, welches möglicherweise eine wichtige Rolle in der Regulierung der Lagerung und/oder Freisetzung von Neurotransmittern spielt, war bereits Gegenstand intensiver Untersuchungen. Aus R. Leube et al., EMBO Journal, Vol. 6 (1987), 3261-3268 ist es bereits bekannt, daß verschiedene Arten von menschlichen kleinzelligen Lungen-Karzinomen durch das Auftreten von Synaptophysin mRNA charakterisiert werden können. Das Synaptophysin selbst, welches ein N-glykosyliertes Protein ist, findet sich üblicherweise in neurosekretorischen Vesikeln, insbesondere in präsynaptischen Vesikeln, sowie in Vesikeln von verschiedenen neuroendokrinen Zellen und zwar sowohl vom neuronalen als auch epithelialen Phänotyp. Die Aminosäuresequenz dieses Proteins und die Nukleotidsequenz der dazugehörigen mRNA ist sowohl für menschliche als auch für Rattenzellen z.B. aus T. Südhof et al., Nucleic Acids Research, Vol. 15, Nr. 22 (1987), 9607 sowie aus T. Südhof et al., Science, Vol. 238 (1987), 1142-1144 sowie aus der schon zuvor zitierten Literaturstelle von R. Leube et al. bekannt. Danach handelt es sich beim Synaptophysin um ein in die Vesikelmembran integriertes Protein, welches über seine Gesamtlänge die Vesikelmembran viermal nähfadenartig durchstößt (Science 238 (1987) 1142-1144, Science 242 (1988) 1050-1052). Darüberhinaus sind auch bereits monoklonale Antikörper bekannt, die gegen Sequenzen der 89 carboxyterminalen Aminosäuren, insbesondere des Ratten-Synaptophysins, gerichtet sind.

Im erfindungsgemäßen Verfahren wird vorzugsweise als Körperflüssigkeit Serum verwendet. Es sind jedoch auch andere Körperflüssigkeiten wie Lungensekrete, Lymphe oder Speichel zur Durchführung des erfindungsgemäßen Verfahrens verwendbar. Die Bestimmung des Synaptophysins in der Körperflüssigkeit erfolgt auf an sich für den Fachmann bekannte Weise. Vorzugsweise wird jedoch das Synaptophysin mit immunologischen Methoden nachgewiesen. Hierbei hat es sich als zweckmäßig erwiesen, Antikörper (als Antiseren, polyklonale oder monoklonale Antikörper) zu verwenden, die gegen die aus der Vesikel-Membran herausragenden Sequenzen des Synaptophysins gerichtet sind. Zweckmäßigerweise werden für das Ausbilden der Antikörper Antigendeterminanten verwendet, deren Aminosäuresequenzen von menschlichen oder vom Ratten-Synaptophysin abgeleitet sind. Besonders wirksame Epitope, die zur Ausbildung von im erfindungsgemäßen Verfahren zu verwendenden Antikörpern geeignet sind, werden durch die Aminosäuresequenzen gebildet, die am Carboxy-terminalen Ende des Synaptophysin-Moleküls liegen. Dies sind insbesondere die 89 Aminosäuren des Carboxy-terminalen Ende des Ratten-Synaptophysins (Aminosäuren 219-307) und die entsprechende 90 Aminosäuren lange Sequenz des menschlichen Synaptophysins (Aminosäuren 207-296).

Als für das erfindungsgemäße Verfahren besonders geeignet haben sich auch Antikörper erwiesen, die gegen das Translations-Produkt des Klon-Fragmentes von pSR[1] oder Bruchstücken davon gerichtet sind. Die Herstellung des Klon-Fragmentes pSR[1] ist bekannt und z.B. von R.E. Leube et al., in Synaptophysin: Molecular Organisation and mRNA-expression as determined from cloned cDNA, EMBO Journal, Vol. 6, Nr. 11, (1987),5. 3261-3268 beschrieben.

Zur Durchführung des erfindungsgemäßen Verfahrens eignen sich sowohl monoklonale als auch polyklonale Antikörper, wobei jedoch monoklonale Antikörper bevorzugt sind. Als besonders zweckmäßig hat es sich erwiesen, zur Durchführung des erfindungsgemäßen Verfahrens den MAK SY38 (Gell 41 (1985) 1017-1028) zu verwenden, der aus der mit der Nummer 89112801 bei der ECACC hinterlegten Zellinie erhältlich ist.

Neben den Antikörpern selbst finden im erfindungsgemäßen Verfahren jedoch auch Antikörper-Bruchstücke Verwendung, welche die hypervariablen Regionen enthalten, wie z.B. die Fab- oder F(ab')$_2$-Fragmente. Diese Fragmente liegen vorzugsweise in immobilisierter Form auf einem Träger gebunden vor und eignen sich besonders zur Durchführung von Enzym- bzw. Radioimmunoassays.

Üblicherweise wird die Bestimmung des Synaptophysins im Serum so durchgeführt, daß man

1. einen Synaptophysin-Antikörper, der vorzugsweise ein MAK ist, an einen Träger, üblicherweise eine Mikrotiterplatte, bindet,

2. anschließend mit der Probe inkubiert, wobei das zu bestimmende Antigen an den Antikörper bzw. dessen Bruchstücke gebunden wird,

3. das gebundene Antigen durch einen üblicherweise identischen, jedoch markierten Synaptophysin-Antikörpers, inkubiert und

4. mit einem chromogenen Substrat eine Nachweisreaktion durchführt und

5. die Absorption des chromogenen Substrates photometrisch mißt.

Es hat sich als vorteilhaft erwiesen, bei der Durchführung des ersten Verfahrensschrittes der Antikörper entweder direkt an den Träger zu binden oder auf eine andere, dem Fachmann bekannte Weise zu immobilisieren, wie z.B. als Biotin-Konjugat an eine mit Streptavidin oder Avidin beschichtete Platte. Bei der Durchführung des Verfahrensschrittes 3 ist es auch möglich, als Detektor AK einen Antikörper zu verwenden, der ein anderes Epitop auf dem Synaptophysin erkennt als der AK des Verfahrensschrittes 1. Ebenso ist es erfindungsgemäß möglich, die Markierung auch mittels radioaktiven, fluoreszierenden oder chemilumineszierenden Substanzen durchzuführen. Enzyme, die sich für eine solche Markierung eignen, sind dem Fachmann bekannt. Als besonders geeignet haben sich jedoch Peroxidase, alkalische Phosphatase und β-Galaktosidase erwiesen.

Die Erfindung wird durch die folgenden Beispiele näher erläutert.

**Beispiel 1**

Nachweis von Synaptophysin

Die wells einer Mikrotiterplatte (Firma Nunc) werden mit 2 μg/ml MAK Sy 38 in Bicarbonatpuffer, pH 9,6, während einer Stunde bei Raumtemperatur beschichtet. Es wird dreimal mit 0,05 % Tween® 20/PBS (8 g/l NaCl, 0,2 g/l KCl, 1,44 g/l Na$_2$HPO$_4$x2H$_2$O, 0,2 g/l KH$_2$PO$_4$) gewaschen und mit 2 % Ovalbumin/PBS (phosphate buffered saline) während einer Stunde bei Raumtemperatur nachbeschichtet. Anschließend wird dreimal mit 0,05 % Tween® in PBS gewaschen.

Die zu bestimmende Probe wird mit PBS verdünnt zugegeben. Es wird 1,5 Stunden bei Raumtemperatur inkubiert und viermal mit 0,05 % Tween® gewaschen. Anschließend wird eine Lösung von einem Konjugat von MAK SY 38 an Peroxidase (Peroxidaseaktivität 150 U/ml) zugegeben, 1,5 Stunden bei Raumtemperatur inkubiert und dreimal mit 0,05 % Tween® PBS gewaschen.

Die Peroxidase-Aktivität wird über die ABTS®-Farbreaktion, nach Zugabe einer Lösung von 1,9 mmol/l ABTS® (2,2″-Azino-di[3-ethyl-benzthiazolin-sulfonsäure(6)]-diammoniumsalz, 100 mmol/l Phosphat/Citratpuffer, pH 4,4, 3,2 mmol/l Natriumperborat), zugegeben und die Extinktion bei 405 nm als Maß für die Analytkonzentration gemessen.

**Beispiel 2**

Mit einer nach dem Beispiel 1 hergestellten Mikrotiterplatte wurden Seren von 52 Patienten untersucht. Das Ergebnis dieser Untersuchung ist der folgenden Tabelle 1 zu entnehmen.

Tabelle 1

| | positiv | untersucht |
|---|---|---|
| 1. Gesunde | 0 | 7 |
| 2. Benigne Krankheiten | 1 | 15 |
| 3. Karzinome | | |
| 3.1. Bronchialkarzinome: | | |
| non-SCLC | 1 | 4 |
| SCLC | 6 | 11 |
| 3.2. andere Karzinome | 1 | 15 |

**Patentansprüche**

1. Verfahren zum Nachweis eines kleinzelligen Bronchial-Karzinoms, **dadurch gekennzeichnet,** daß man Synaptophysin, seine Oligomere und/oder seine Bruchstücke in Körperflüssigkeiten bestimmt.

2. Verfahren nach Anspruch 1, **dadurch gekennzeichnet,** daß man als Körperflüssigkeit Serum verwendet.

3. Verfahren nach einem der vorhergehenden Ansprüche, **dadurch gekennzeichnet,** daß man zur Bestimmung Antikörper verwendet, die gegen die aus der Zellmembran herausragenden Sequenzen des Synaptophysins gerichtet sind.

4. Verfahren nach Anspruch 3, **dadurch gekennzeichnet,** daß man Antikörper verwendet, die gegen das carboxyterminale Ende gerichtet sind.

5. Verfahren nach einem der vorhergehenden Ansprüche, **dadurch gekennzeichnet,** daß man Antikörper verwendet, die gegen das Translationsprodukt des Klonfragmentes pSR[1] oder Bruchstücke davon gerichtet sind.

6. Verfahren nach einem der vorhergehenden Ansprüche, **dadurch gekennzeichnet,** daß man Antikörper verwendet, die gegen die Aminosäure-Sequenz 207 bis 296 des carboxyterminalen Endes von Humansynaptophysin gerichtet sind.

7. Verfahren nach einem der vorhergehenden Ansprüche, **dadurch gekennzeichnet,** daß man Antikörper verwendet, die gegen die Amino-Säure-Sequenz 219-307 des carboxyterminalen Endes des Ratten-Synaptophysins gerichtet sind.

8. Verfahren nach einem der vorhergehenden Ansprüche, **dadurch gekennzeichnet,** daß man Antikörper verwendet, die gegen die letzten 90 Aminosäuren des carboxyterminalen Endes des Human-Synaptophysins gerichtet sind.

9. Verfahren nach einem der vorhergehenden Ansprüche, **dadurch gekennzeichnet,** daß man den aus der Zellinie ECACC 89112801 erhältlichen monoklonalen Antikörper SY38 verwendet.

10. Verfahren nach einem der vorhergehenden Ansprüche, **dadurch gekennzeichnet,** daß man Fab und/oder F(ab')$_2$ verwendet.

## Claims

1. Method for the detection of a small cell lung carcinoma,
**wherein**
synaptophysin, its oligomers and/or its fragments are determined in body fluids.

2. Method as claimed in claim 1,
**wherein**
serum is used as the body fluid.

3. Method as claimed in one of the previous claims,
**wherein**
antibodies are used for the determination which are directed against the sequences of synaptophysin which protrude from the cell membrane.

4. Method as claimed in claim 3,
**wherein**
antibodies are used which are directed against the carboxy-terminal end.

5. Method as claimed in one of the previous claims,
**wherein**
antibodies are used which are directed against the translation product of the clone fragment pSR[1] or fragments thereof.

6. Method as claimed in one of the previous claims,
**wherein**
antibodies are used which are directed against the amino acid sequence 207 to 296 of the carboxy-terminal end of human synaptophysin.

7. Method as claimed in one of the previous claims,
**wherein**
antibodies are used which are directed against the amino acid sequence 219-307 of the carboxy-terminal end of rat synaptophysin.

8. Method as claimed in one of the previous claims,
**wherein**
antibodies are used which are directed against the last 90 amino acids of the carboxy-terminal end of human synaptophysin.

9. Method as claimed in one of the previous claims,
**wherein**
the monoclonal antibody SY38 derived from the cell line ECACC 89112801 is used.

10. Method as claimed in one of the previous claims,
**wherein**
Fab and/or $F(ab')_2$ is used.


## Revendications

1. Procédé de mise en évidence d'un cancer bronchique à petites cellules, caractérisé en ce que l'on détermine la synaptophysine, ses oligomères et/ou ses fragments dans les liquides de l'organisme.

2. Procédé selon la revendication 1, caractérisé en ce que l'on utilise le sérum comme liquide de l'organisme.

3. Procédé selon l'une des revendications précédentes, caractérisé en ce que, pour la détermination, on utilise des anticorps qui sont dirigés contre les séquences de la synaptophysine qui font saillie de la membrane cellulaire.

4. Procédé selon la revendication 3, caractérisé en ce que l'on utilise des anticorps qui sont dirigés contre l'extrémité carboxy-terminale.

5. Procédé selon l'une des revendications précédentes, caractérisé en ce que l'on utilise des anticorps qui sont dirigés contre le produit de traduction du fragment de clone pSR[1] ou de fragments de celui-ci.

6. Procédé selon l'une des revendications précédentes, caractérisé en ce que l'on utilise des anticorps qui sont dirigés contre la séquence d'acides aminés 207 à 296 de l'extrémité carboxy-terminale de la synaptophysine humaine.

7. Procédé selon l'une des revendications précédentes, caractérisé en ce que l'on utilise des anticorps qui sont dirigés contre la séquence d'acides aminés 219-307 de l'extrémité carboxy-terminale de la synaptophysine de rat.

8. Procédé selon l'une des revendications précédentes, caractérisé en ce que l'on utilise des anticorps qui sont dirigés contre les 90 derniers acides aminés de l'extrémité carboxy-terminale de la synaptophysine humaine.

9. Procédé selon l'une des revendications précédentes, caractérisé en ce que l'on utilise l'anticorps monoclonal SY38 qui peut être obtenu à partir de la lignée cellulaire ECACC 89112801.

10. Procédé selon l'une des revendications précédentes, caractérisé en ce que l'on utilise Fab et/ou $F(ab')_2$.